# EUROPEAN PATENT APPLICATION

(11) **EP 2 044 930 A1**
(43) Date of publication of application: **08.04.2009**
(21) Application number: 07117649.9
(22) Date of filing: 01.10.2007
(51) Int. Cl.: A61K 9/10, A61K 47/14, A61K 47/44, A61P 3/04

(54) **Composition for controlling lipase catalyzed reactions**

(71) Applicant: Nestec S.A., 1800 Vevey (CH)
(72) Inventor: Reis, Pedro, Houston, TX 77030-3411 (US); Leser, Martin, ch-1053 Bretigny-s/Morrens (CH); Watzke, Heribert Johann, ch-1012 Lausanne (CH)
(74) Representative: Marquardt, Ulf

(57) **Abstract**

The present invention relates to the field of lipolysis mediated by lipases. In particular the present invention relates to the modulation of lipase activity by regulation of the composition of the interface between a hydrophobic and a hydrophilic phase. More particularly the present invention relates to a the use of a formulation comprising at least one surfactant with an interfacial pressure that is sufficiently high to control the access of lipase substrates to the interface between a lipophilic phase and a hydrophilic phase to regulate lipolysis and to a composition comprising at least one oil and enriched with at least one surfactant wherein the surfactant is non-cleavable by at least one lipase, has a higher affinity to the interface between the hydrophilic and lipophilic phase than the at least one lipid and is present in a weight ratio to the at least one lipid of about 1:1000 - 100:1.

## Description

The present invention relates to the field of lipolysis mediated by lipases. In particular the present invention relates to the modulation of lipase activity by controlling the access of lipase substrates to the interface between a lipophilic phase and a hydrophilic phase. More particularly the present invention relates to a use in accordance with claim 1 and a composition in accordance with claim 30.

Lipases are a group of enzymes existing in the gastro intestinal tract. Their main function is to hydrolyse, i.e., digest, dietary fats, e.g., triglycerides or diglycerides, allowing their assimilation by the body. Lipases can also be used to hydrolyse non-triglyceride fats, such as p-nitrophenyl palmitate or decylchloroacetate or others. The generated lipid molecules are mainly free fatty acids and monoglycerides (when using triglycerides). An uncontrolled uptake of fats during digestion can lead to different phenomena: a high uptake of these fat components can lead to severe health problems, such as obesity, arterioscleroses or related disorders, and a low fat uptake can lead to health problems like malabsorption and malnutrition.

Several strategies are presently discussed in the literature that aim at decreasing the assimilation of dietary fats by the body during digestion.

One strategy to achieve this goal is by replacing the fat in food products by fat replacers, (e.g. US2001003119AA; US2003215556AA) or other fat-mimicking ingredients that are not hydrolysed by lipases (e.g. modified starch, dextrins, oat fiber, polydextrose, gums). Fat replacers, however, usually result in a slightly different mouthfeel and/or taste of the final product.

Another strategy to decrease the uptake of fat is by adding a component which acts as an inhibitor of the lipases. An example is Orlistat^{®}, which is a phosphonate molecule that is taken as a supplement and irreversibly binds to the catalytic site of lipases. Therefore, hydrolysis of triglycerides is decreased. However, undesirable side effects (e.g., laxative effect and loss of lipophilic vitamins absorption) might occur.

A third strategy deals with the addition of components which limit the assimilation of dietary fats by physically entrapping the fat or fat globules. US6214349 describes a non-digestible dietary fiber- emulsifier mixture that in this way reduces fat uptake during digestion. Usually, however, these products are relatively expensive and difficult to produce. They may further have an influence on the texture of the final product.

A fourth strategy is to add ingredients that induce fat burning. US 6762203 discloses a composition based on a diglyceride/monoglyceride mixture which is shown to be effective in this regard. The addition of such agents, however, puts a pressure on the metabolism of an organism, which may be advisable to avoid in certain cases.

It was thus one object of the present invention to overcome the disadvantages of the approaches of the prior art and to provide a new alternative approach to decrease fat assimilation by the body in humans or animals during digestion.

This new approach uses an agent that is capable of controlling the molecular composition of the water-oil interface, which is the location of action of the lipase reaction. The water-oil interface is mainly composed of (i) the lipase (and co-lipase) and (ii) substrates for the lipase ( e.g., triglycerides (abbreviated as TAG) and/or diglycerides (abbreviated as DAG). If the fat is present in an emulsified form, the water-oil interface contains also emulsifiers which are used during the emulsification process to produce a physically stable fat emulsion product.

The present inventors were surprised to discover that fat biocatalysis can be hindered by using interfacially active molecules at the interface, which decrease substrate accessibility (e.g., triglycerides or diglycerides) to lipase.

This control of the water-oil-interface composition in the present invention may be realised by adding a surfactant (lipid) which is more surface active than the substrates of the lipase (TAG or DAG) to a fat composition. As a consequence, this surfactant excludes the lipase substrates from the interface which in turn leads to a reduction (control) of the access of the lipase to its substrate. The exclusion of the substrate from the interface reduces the lipolysis activity of lipases.

The degree of substrate exclusion may be controlled, e.g., by (i) the choice (type) of added surfactant. Important aspects are its surface activity with respect to the surface activity of the lipase substrate and/or whether or not the surfactant is itself hydrolysable by lipases or not) and by (ii) the surfactant concentration in the product, and by this the surfactant concentration at the interface between the hydrophilic and the hydrophobic phase.

The surfactant may either be added to the product prior to digestion or can be produced in-situ during digestion, e.g. by the action of esterases, proteases or lipases that are present in the gastro-intestinal tract.

This ability to control the water-oil interface composition may be used to regulate fat assimilation during digestion, i.e., decreasing fat assimilation in subjects suffering from obesity and related health problems, or to influence satiety and/or satiation.

Without wishing to be bound by theory the present inventors believe that the adsorption of the surfactant to the water-oil interface is able to at least partially block the lipase substrates, e.g. triglycerides or diglycerides, from accessing the active site of the lipase. This leads to a reduction of the extent and kinetics of triglyceride (or diglyceride) hydrolysis. The surfactant itself may be hydrolysable or non-hydrolysable by lipases.

It is essential to the present invention that the surfactant used shows a higher surface activity than the lipase substrates such as, e.g., TAG or DAG, so that it expels the substrate molecules from the interface thus reducing their contact with the lipase molecules and - as a consequence - reducing the hydrolysis action of lipase on the oil substrates.

According to the present invention a fat containing diet may be enriched with surfactants that are able to co-adsorb with the lipase to the water-oil interface during the digestion process. This reduces the amount of TAG and/or DAG molecules at the interface, and by this the molecular contact with the lipase molecules at the interface is reduced. The TAG- or DAG-hydrolysing activity of lipases is reduced. Instead, the more surface active hydrolysable surfactant is digested, or, in case the surfactant itself is non-hydrolysable, the lipase might synthesize diglycerides and/or triglycerides instead starting from the surfactant and other surface active molecules present at the water-oil interface, such as fatty acids.

Consequently, one embodiment of the present invention is the use of a formulation comprising at least one surfactant with an interfacial pressure that is sufficiently high to control the access of lipase substrates to the interface between a lipophilic phase and a hydrophilic phase to regulate lipolysis.

The at least one surfactant is, hence, capable of at least partially replacing lipase substrates from an interface between a lipophilic phase and a hydrophilic phase. Consequently, the present invention relates also to the use of a formulation comprising at least one surfactant to at least partially replace lipase substrates from interface between a lipophilic phase and a hydrophilic phase.

A further embodiment of the present invention is the use of a formulation comprising at least one surfactant with an interfacial pressure that is sufficiently high to at least partially replace lipase substrates from an interface between a lipophilic phase and a hydrophilic phase to regulate lipid lipolysis.

"Interfacial pressure" (IP) means the interfacial tension of pure lipophilic / hydrophilic phases (Io) - interfacial tension of lipophilic / hydrophilic phases with adsorbed material at the interface (I). Consequently, IP = Io- I.
For example in a model system the interfacial pressure may be calculated as the interfacial tension of lipase substrate in phosphate buffer decane interface - interfacial tension of lipase substrate and surfactant in phosphate buffer decane interface.

"Lipolysis" is to be understood as an interfacial reaction of lipases with a substrate and comprises hydrolysis of lipids, synthesis of lipids, ester formation, ester cleavage, amid formation, amide cleavage, inter esterification.

"Lipase substrates" comprise molecules containing acyl-groups and are preferably the lipids described herein.

Digestion is to be understood for the purpose of the present invention as a lipolysis yield higher than 90% at physiological conditions after 3 hours of reaction time.

In one preferred embodiment of the present invention, lipolysis is lipid digestion.

The regulation of lipid digestion according to the present invention may in turn serve several purposes. One preferred purpose is the treatment and/or prevention of metabolic syndromes and/or obesity.

The formulation used in the present invention may - in its simplest form - consist of one surfactant with a surface activity that is sufficiently high to at least partially replace lipase substrates from an interface between a lipophilic phase and a hydrophilic phase. It may equally well comprise more than one surfactant with a surface activity as described above. Different surfactants may be used in a formulation to fine tune the overall surface activity of the formulation to specifically adapt the formulation for a specific application.

The formulation used in the present invention may also comprise a lipophilic compound and/or a hydrophilic compound. At least one surfactant as described above may be dissolved in either the lipophilic and/or the hydrophilic phase. Preferably, the formulation of the present invention comprises both, a lipophilic and a hydrophilic phase.

The formulation of the present invention might be intended to be added to a foodstuff before its consumption. It might also be a foodstuff itself. Equally well the formulation might be foreseen to be consumed before, during and/or after a meal to regulate the digestion of the fats that are present within a meal. In this case it is preferred if the formulation is used before a meal.

In one embodiment of the present invention the formulation used in the present invention may comprise a lipophilic phase and a hydrophilic phase and may be present in the form of an emulsion. An emulsion is a mixture of two immiscible substances. One substance, the dispersed phase, is dispersed in the other, the continuous phase. Examples of emulsions include butter and margarine, espresso or mayonnaise. Emulsions have the advantage that the surfactant is well distributed throughout the emulsion, primarily at the numerous interfaces between hydrophilic and lipophilic phases. This equal distribution allows a good dosability and an equal distribution in the body after ingestion, allowing an optimal effectiveness. Such an emulsion may have an average particle diameter of 5 nm-100 µm. The emulsion may be a micro emulsion. In this case the particle size may range from about 5nm to 500nm. Micro emulsions have the advantage that they exhibit a very high stability. Normal emulsions may have a particle size of about 1µm - 100 µm. Normal emulsions have the advantage that their preparation is simpler, requires less energy and - consequently - is more cost efficient.

A stabilizer may be used in the framework of the present invention. Such a stabilizer may be a stabilizer for emulsions. It may also be, e.g., an antioxidant to stabilize valuable nutritional oils.

Preferably, the lipophilic phase comprises at least one lipid.

Lipids comprise for the purpose of the present invention fatty acids and their derivatives, such as mono-, di-, triglycerides and phospholipids, as well as other fat soluble sterol molecules such as cholesterol.

Adding lipids directly to the formulation has the advantage that lipase is confronted after consumption of the formulation simultaneously with surfactant and lipid, so that the effect of the surfactant is maximal when it is needed most. The lipid may preferably be a nutritionally valuable oil or an oil composition.

Generally, the oil may be selected from the group consisting of triglycerides, fatty acid derivatives, such as fatty acid amides, and mixtures thereof.

The lipid or oil used in the formulation can be either a vegetable fat or oil or an animal fat or oil or a mixture thereof. The vegetable fat or oil is preferably taken from the group consisting of soy oil, corn oil, rapeseed oil, sunflower oil, palmolein, alone or in mixture. In a preferred composition, the rapeseed oil is canola oil. In the case of animal oil or fat, the source is preferably milk fat or tallow. The lipid or oil used in the formulation may further be a wax or an essential oil comprising an ester group.

The present invention is also applicable to the regulation of the lipolysis of esterified compounds in general, in particular of esterified food compounds.

The lipid or oil source may comprise long chain triglycerides (LCTs) and medium chain triglycerides (MCTs).

MCTs can be a mixture of C6-C12. For example, MCTs can be a mixture of C6:0(1-2%), C8:0(65-75%), C10:0(25-30%), and C12:0(1-2%). In an embodiment, the MCTs comprise 20% of the lipid or oil source and LCTs comprise 80% of the lipid or oil source.

The use of MCTs aids in digestion. Digestion of MCTs may be easier than LCTs in that LCTs are digested by various lipases; in contrast to LCTs, pancreatic lipase is not essential to digestion of MCTs. Additionally, absorption of MCTs is faster as compared to LCTs. LCTs require incorporation into chylomicron by intestinal mucosal cells. Similarly, transport of MCTs is via portal circulation directly to the liver whereas LCTs are transported via lymphatics and systemic circulation before finally ending up in the liver. LCTs are oxidized more slowly requiring carnitine for entry into the mitochondria. The source of MCTs can comprise fractionated coconut oil.

Preferably, the LCTs are provided as canola oil, olive oil, and hi-oleic safflower oil. Although other oils can be used such as, e.g., soy oil, high-oleic sunflower oil, or any oil rich in mono-unsaturated fatty acid (MUFA). These oils not only provide linoleic acid, an essential fatty acid, but also provide n-3 fatty acids. Linolenic acid, the predominate n-3 fatty acids supplied by these oils, may serve as a precursor to other n-3 fatty acids which have anti-inflammatory activity. Preferably, at least 4%-10%, by calories, essential fatty acids are provided by the composition of the present invention.

Preferably, in an embodiment, the ratio of n-6:n-3 fatty acids is approximately 4. However, other ratios can be used with preferably the ratio of n-6:n-3 being 2 to 10. This lower ratio may improve the immune response.

Additionally, the fat source may comprise approximately 40% to about 70% of the total calories as mono-unsaturated fatty acids (MUFA). In a preferred embodiment, the MUFA content of the fat is approximately 58% by caloric content. This higher level of MUFA as part of a high fat/moderate carbohydrate diet provides lower serum lipids than a lower fat diet that does not contain a significant amount of MUFA.

In a preferred embodiment of the present invention is the at least one surfactant at least partially located at the oil-water interface. Even more preferred is that at least one surfactant is primarily located at the oil-water interface. Still more preferred is that at least one surfactant is almost exclusively, e.g., > 95%, located at the oil-water interface.

The surfactant may also have a higher affinity to the interface between the hydrophilic and lipophilic phase than the at least one lipid and may furthermore be non-cleavable by lipases of the body.

"Non-cleavable" means for the purpose of the present invention that a compound is hydrolyzed less than 10 % by the action of lipases in 1 h of reaction time at physiological conditions.

A "lipase" is a molecule able to, e.g., mediate hydrolysis of acyl-ester bounds of water insoluble and amphiphilic molecules at physiological conditions.

Preferably, the lipase may be selected from the group consisting of gastro-intestinal lipases, in particular lingual lipase, gastric lipase and pancreatic lipase or mixtures thereof.

Generally any surfactant with an interfacial pressure that is sufficiently high to control the access of lipase substrates to the interface between a lipophilic phase and a hydrophilic phase is applicable for the purpose of the present invention. Preferably, since the formulation is intended for consumption, the surfactant is a food grade or pharmaceutical grade surfactant. The surfactant may be a mono- or di-acyl glyceride, and its Sn-2 position may be acylated. The fatty acid residues of the surfactants are not particularly limited. However, e.g., for food applications it is preferred that they have a chain length of between 8 and 22 carbon atoms. The fatty acid residue may be selected from the group consisting of saturated and polyunsaturated fatty acid residues.

The surfactant may be selected from the group consisting of low molecular weight surfactants or high molecular weight surfactants. A low molecular weight surfactant may have a molecular weight of less than 2000 Dalton, while a high molecular surfactant may have a molecular weight of more than 2000 Dalton.

The surfactant may be preferentially selected from the group consisting of low molecular weight surfactants, e.g., myristic acid, oleic acid, lauric acid, stearic acid, palmitic acid, PEG 1-4 stearate, PEG 2-4 oleate, PEG-4 dilaurate, PEG-4 dioleate, PEG-4 distearate, PEG-6 dioleate, PEG-6 distearate, PEG-8-dioleate, PEG-3-16 castor oil, PEG 5-10 hydrogenated castor oil, PEG 6-20 corn oil, PEG 6-20 almond oil, PEG-6 olive oil, PEG-6 peanut oil, PEG-6 palm kernel oil, PEG-6 hydrogenated palm kernel oil, PEG-4 capric/caprylic triglyceride, mono, di, tri, tetraesters of vegetable oil and sorbitol, pentaerythrityl di, tetra stearate, isostearate, oleate, caprylate or caprate, polyglyceryl-3 dioleate, stearate, or isostearate, plyglyceryl 4-10 pentaoleate, polyglyceryl 2-4 oleate, stearate, or isostearate, polyglyceryl 4-10 pentaoleate, polyglycewryl-3 dioleate, polyglyceryl-6 dioleate, polyglyceryl-10 trioleate, polyglyceryl-3 distearate propylene glycol mono- or diesters of C6 to C20 fatty acid, monoglycerides of C6 to C20 fatty acid, lactic acid derivatives of monoglycerides, lactic acid dericatives of diglycerides, diacetyl tartaric ester of monoglycerides, triglycerol monostearate cholesterol, phytosterol, PEG 5-20 soya sterol, PEG-6 sorbitan tetra, hexasterarate, PEG-6 sorbitan tetraoleate, sorbitan monolaurate, sorbitan monopalmitate, sorbitan mono trioleate, sorbitan mono and tristearate, sorbitan monoisostearate, sorbitan sesquioleate, sorbitan sesquistearate, PEG-2-5 oleyl ether, POE 2-4 lauryl ether, PEG-2 cetyl ether, PEG-2 stearyl ether, sucrose ester, sucrose distearate, sucrose dipalmitate, ethyl oleate, isopropyl myristate, isopropyl palmitate, ethyl linoleate, isopropyl linoleate, poloxamers, phospolipids, lyso-phospholipids, lecithins, cephalins, oat lipids, glycolipids, and amphiphilic lipids from plants; or high molecular weight surfactants, e.g., proteins from plant or animal origin, or mixtures thereof.

The amount of surfactant used in the formulation of the present invention is not particularly limited. Any effective amount can be used. However, preferably, the surfactant is present in an amount of 0.1 - 99 weight-%, preferably 5-80 weight-%, more preferably 10-70 weight-%, even more preferred 15-60 weight-%, most preferred 20-50 weight-% of the formulation.

The present invention comprises the use of a formulation as described above for the preparation of a composition. The use of the present invention further comprises medical uses and non-medical uses. Consequently, the composition may be a pharmaceutical composition or a food product.

If the composition is a food product, it is preferably a lipid containing product. Particular preferred products are food products such as, e.g., mayonnaises, salad dressings, milk based products, coffee creamers, pre-cooked meals, food powders, food additives. Particular preferred pharmaceutical products may be creams for topical applications, shampoos, delivery systems, compositions for enteral application.

The surfactant may be present in such a composition in an amount of 0.1 - 50 weight-% preferably 2-45 weight-%, more preferably 4-40 weight-%, even more preferred 8-35 weight-%, most preferred 10-30 weight-% of the of the composition.

The interfacial pressure of the surfactant may be about 5-50 mN/m, preferably 30-50 mN/m.

The use of the present invention may be carried out in a body of an animal, preferably in the body of a mammal, in particular in the body of a human or a pet. In this case the present invention relates to the use of a formulation comprising at least one surfactant with an interfacial pressure that is sufficiently high to control the access of lipase substrates to the interface between a lipophilic phase and a hydrophilic phase to prepare a composition to regulate lipolysis.

The formulation and/or composition of the present invention may be used specifically to expel lipase substrates from an oil-water interface in the stomach, duodenum, ileum and/or jejunum.

The subject matter of the present invention may thus be used to reduce lipid digestion, to retard fat digestion, to decrease energy release from ingested food, to prolong the feeling of satiety and/or to improve satiation.

Consequently, the present invention relates to the use of a formulation comprising at least one surfactant with an interfacial pressure that is sufficiently high to control the access of lipase substrates to the interface between a lipophilic phase and a hydrophilic phase to prepare a composition to reduce lipid digestion, to retard fat digestion, to decrease energy release from ingested food, to prolong the feeling of satiety and/or to improve satiation.

Satiety is defined as the feeling of having eaten enough.

Satiation is defined as the condition of not being hungry.

Those of skill in the art will understand that all features of the present invention as described above with respect to the use of the present invention or with respect to the formulation used apply vice versa to the composition of the present invention unless otherwise indicated. Similarly, all features mentioned with respect to the composition apply to the formulation and its use.

Consequently, the present invention relates to a composition comprising at least one surfactant with an interfacial pressure that is sufficiently high to at least partially replace lipase substrates from an interface between a lipophilic phase and a hydrophilic phase.

In the framework of a preferred embodiment the present invention relates to a composition comprising at least one oil and enriched with at least one surfactant wherein the surfactant is non-cleavable by at least one lipase, has a higher affinity to the interface between the hydrophilic and lipophilic phase than the at least one lipid and is present in a weight ratio to the at least one lipid of about 1:1000 - 100:1.

"Enriched" means for the purpose of the present invention the incorporation of a molecule at concentrations higher than naturally occurring.

"Having a higher affinity to the interface" between the hydrophilic and lipophilic phase than the at least one lipid means for the purpose of the present invention that the surfactant is more amphiphilic than the lipase substrate.

The composition of the present invention may furthermore have all features described above for the formulation and its use unless otherwise indicated.

Those skilled in the art will understand that they can freely combine all features of the present invention described herein without departing from its scope as disclosed by this specification.

Further advantages and embodiments of the present invention will be apparent from the following figures and examples.
Figure 1 shows an experimental setup of an example of a gastro-intestinal model from TNO adopted for analysing the effect of surface active molecules on the hydrolysis of Tricaprylin (TC8)
Figure 2 shows a caprylic acid (C8) profile obtained after adsorption in the ileum and jejunum of Tricaprylin, with / without Sn-2 Monopalmitin (Sn2-M16).
Figure 3 shows a caprylic acid (C8) profile obtained in the lumen of stomach after feeding TIM with Tricaprylin, with / without Sn-2 Monopalmitin (Sn2-M16), betalactoglobulin (BLG) and Lysophosphotidylcholine (LysPC).
Figure 4 shows a caprylic acid (C8) profile obtained in the lumen of duodenum, jejunum and efflux after feeding TIM with Tricaprylin, with / without Sn-2 Monopalmitin (Sn2-M16).
Figure 5 shows a chromatogram from GC-FID, comparing the caprylic acid profile of 2 TIM samples analyzed in the ileum after 2h of digestion. Sample a) presence of sn-2 monopalmitin during digestion; sample b) control - no added sn-2 monopalmitin
Figure 6 shows the effect of different monoglycerides (1,3 E-3M) on lipolytic hydrolysis of p-nitrophenylpalmitate.
Figure 7 shows the effect of different monoglycerides (1,3 E-3M) on lipolytic hydrolysis of tricaprylin.
Figure 8 shows an example of a pendant Drop technique. The interfacial tension in a decane-buffer solution is depicted as a function of titrated (added) Sn-2 monoarachidin (non-hydrolysable surfactant).
Figure 9 shows a comparison of the interfacial tension properties of caprylic acid (C8), monocaprylin (MC8) and dicaprylin (DC8) on a buffer / decane interface.
Figure 10 shows a lipolysis of p-nitrophenylpalmitate in the presence of a cleavable surfactant (Sn 1/3 Monolaurin) or a non hydrolysable surfactant (Sn2 Monolaurin)

### Example 1 shows the reduction of the lipolytic degradation of a triglyceride oil in the presence of surface active molecules in a feeding system

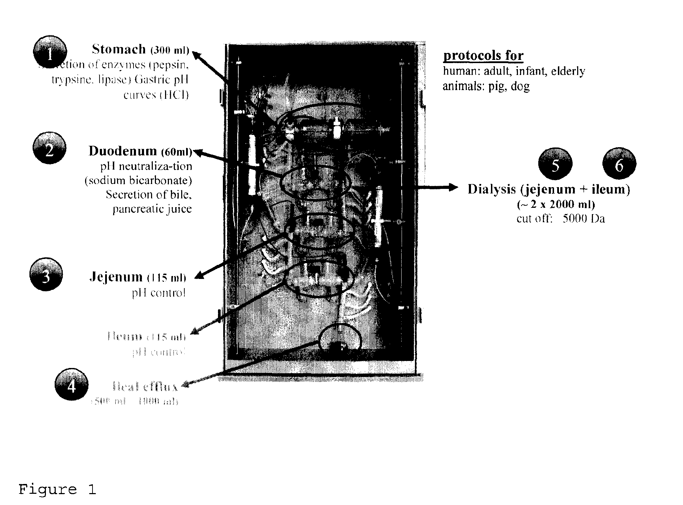

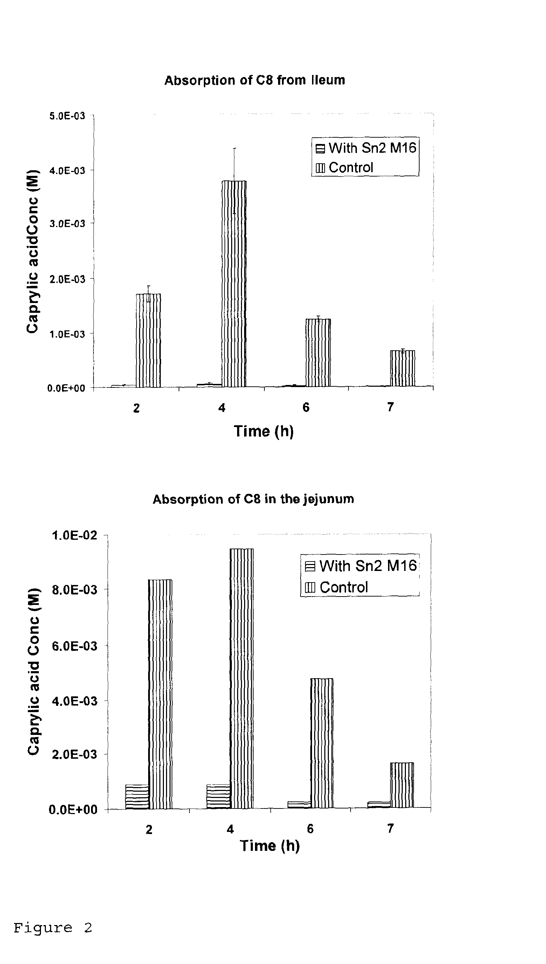

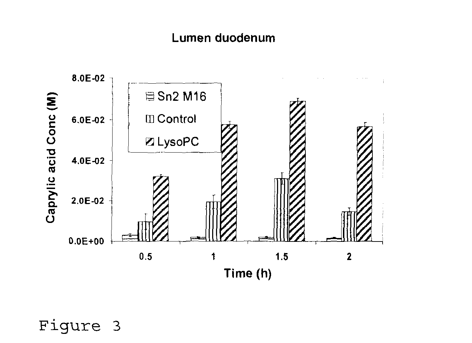

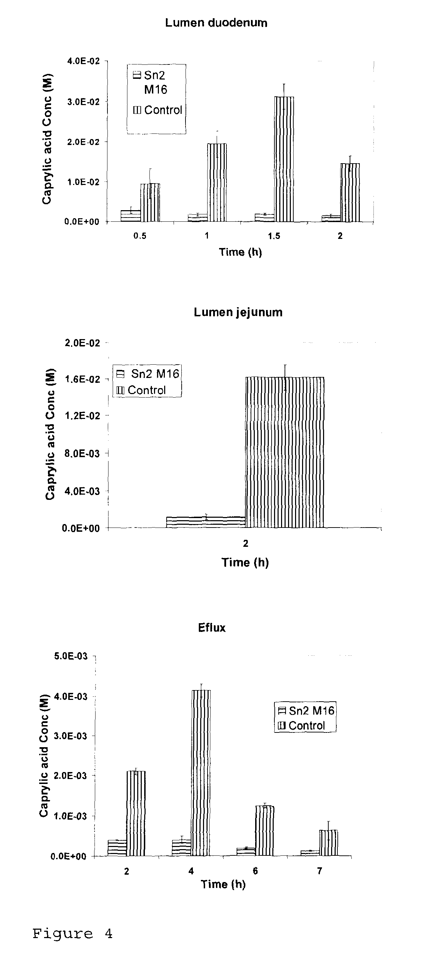

8 ml Tricaprylin containing 2.0 g Sn2-Monopalmitin (non-cleavable surfactant) was mixed with 200 ml phosphate buffer pH 5.5 and 20 mg/ml starch (emulsifier) using an Ultra Turrax for 2 minutes, and subsequent sonification for 10 minutes. The reference emulsion system contains the same amount of tricaprylin and starch, however, no Sn2-monopalmitin. Model digestion runs of the 2 samples were performed using a gastro-intestinal model system provided by TNO. Physiological conditions (e.g. temperature, enzyme type and load, bile salt concentration, pH) were predefined via the TIM programming system. The hydrolysis of Tricaprylin was followed by monitoring the caprylic acid release after passing the different gastro-intestinal compartments. Figure 1 shows the used TIM setup up from TNO and the different compartments representing the stomach, duodenum, jejunum and ileum. The extraction of the generated fatty acids after passing the different compartments was performed by taking 100 ul of the reaction mixture, adding 900 ul chloroform together with 100 ul Chloridric acid 1 M. The samples were vortexed and centrifuged at 5000 rpm for 10 min. 500 ul of the supernatant was taken and directly injected into a GC-FID. 3 samples were analysed for each reaction time at each gastro-intestinal compartment. Figure 2 shows the appearance of the Caprylic acid (C8) profile after passing through the ileum and jejunum in the presence and absence of the surfactant Sn-2 Monopalmitin (Sn2-M16). Whereas in the absence of the Sn2-M16 significant amounts of caprylic acid can be detected (is the results of the lipolytic degradation of the tricaprylin), almost no caprylic acid is detected in the presence of the Sn-2-M16 (a non-hydrolysable surfactant). A similar result is obtained when measuring caprylic acid content in the lumen of the stomach, duodenum, jejunum and in the efflux at the end of the digestion path (see Figure 3, 4 and 5). This shows that addition of the non-cleavable surfactant (Sn2-M16) to the triglyceride oil allows to reduce the lipolytic fat hydrolysis and absorption.
Additional experiments were performed where sn-2 monopalmitin was replaced Lysophosphotidilcholine (LysoPC). The effect of LysoPC on fat gastric digestion were studied. As can be seen on Figure 3, LysoPC also had a considerable effect in decreasing gastric lipolysis of triglycerides.

### Example 2 shows the controlled lipolytic hydrolysis of p-nitrophenypalmitate or tricaprylin as a function of the addition of non-hydrolysable and hydrolysable surfactants, having different chain lengths.

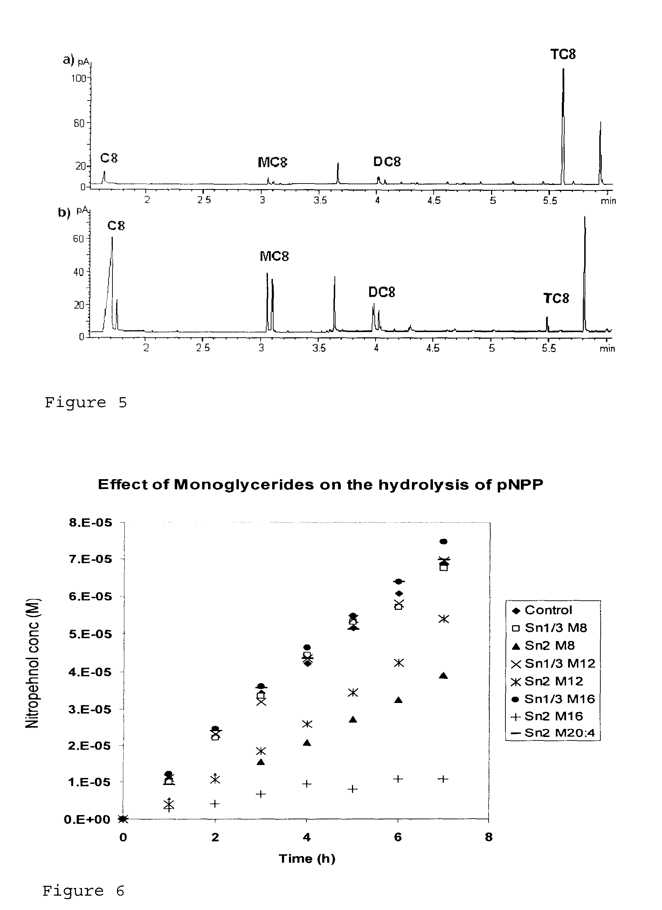

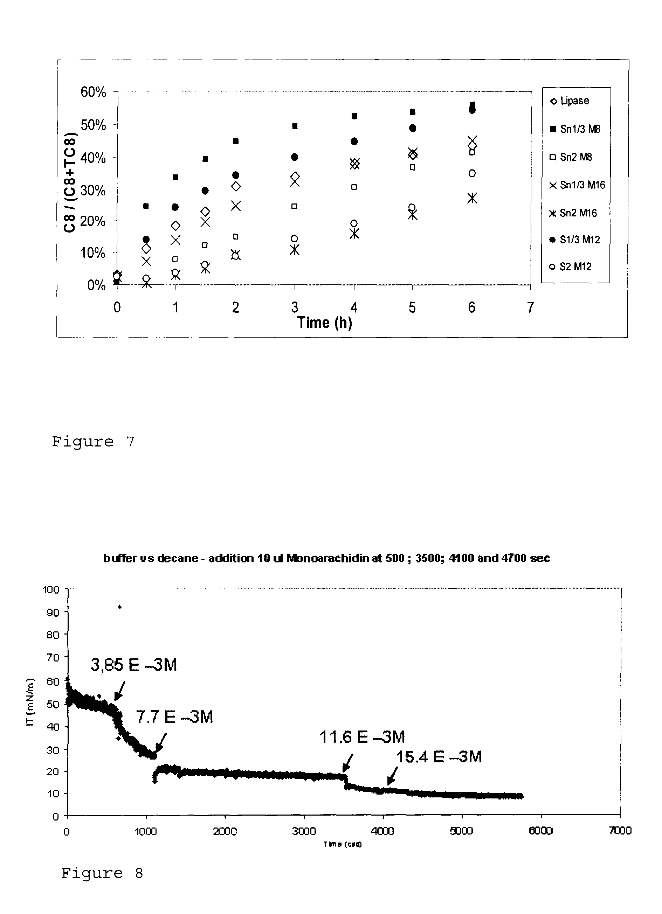

A biphasic system was prepared in glass test tubes with the following composition:
○ Upper oil phase is composed of 7 ml decane in the absence (control) or presence of (1,3 E-3M) monoglyceride and 20 ul tricaprylin or p-nitrophenypalmitate (2E-4M)
○ Aqueous phase is composed of 2 ml phosphate buffer, pH 5.5 containing 3,3 E-6 M *Rhizomucor miehei* lipase. The caprylic acid generation in the presence and absence of monoglycerides (surfactant) is monitored by GC-FID. 100 ul of the upper oil phase are taken and added to 900 ul Chloroform before direct injection into the GC. The nitrophenol generation is followed by spectroscopic analysis at 410 nm of the aqueous phase. Figures 7 and 8 show the amount of caprylic acid formed after the lipolytic hydrolysis of the p-nitropheylpalmitate and tricaprylin in the presence of Sn1 (hydrolysable surfactants) or Sn2 (non-hydrolysable) monoglycerides. Whereas the kinetics of the lipolytic caprylic acid formation in the presence of the hyrolysable surfactants Sn1 M8 or Sn1 M12 is similar to the kinetics in the absence of surfactants, the kinetics of caprylic acid formation is reduced in the presence of the non-hydrolysable surfactants Sn2-M8, Sn2-M12 or Sn2-M16. Figure 5 shows a similar effect using another oil substrate for the lipase. Again in the presence of the hydrolysable surfactants Sn1-M8, Sn1-M12 or Sn1-M16, the kinetics of nitrophenol formation is quite similar to the observations made in the reference system (no surfactant added). However, in the presence of the non-hydrolysable surfactant Sn2-M8, Sn2-M12 or Sn2-M16 a significant reduction in the nitrophenol formation kinetics is observed. Note that when using the non-hydrolysable surfactant Sn2M20:4, the observed kinetics of nitrophenol formation is again similar to the kinetics observed in the system without monoglyceride added. This is an indication that with the used monoglyceride concentration (the concentration was constant at 1.3 E-3 M) not enough surfactant is adsorbing to the water-oil interface, since the Sn2M20:4 is too lipohilic and partitions more into the bulk oil than to the interface.

### Example 3 shows the use of interfacial tension measurements to demonstrate how the pendant drop technique is used to define the surface activity of the added non-hydrolysable surfactants.

The effect of the addition of the Sn-2 monoglyceride monoarachidin, a non-hydrolysable surfactant, is shown in Figure 8. It induces a decrease of the measured interfacial tension between the buffer and the decane solution, showing that the surfactant adsorbs to the water-decane interface.

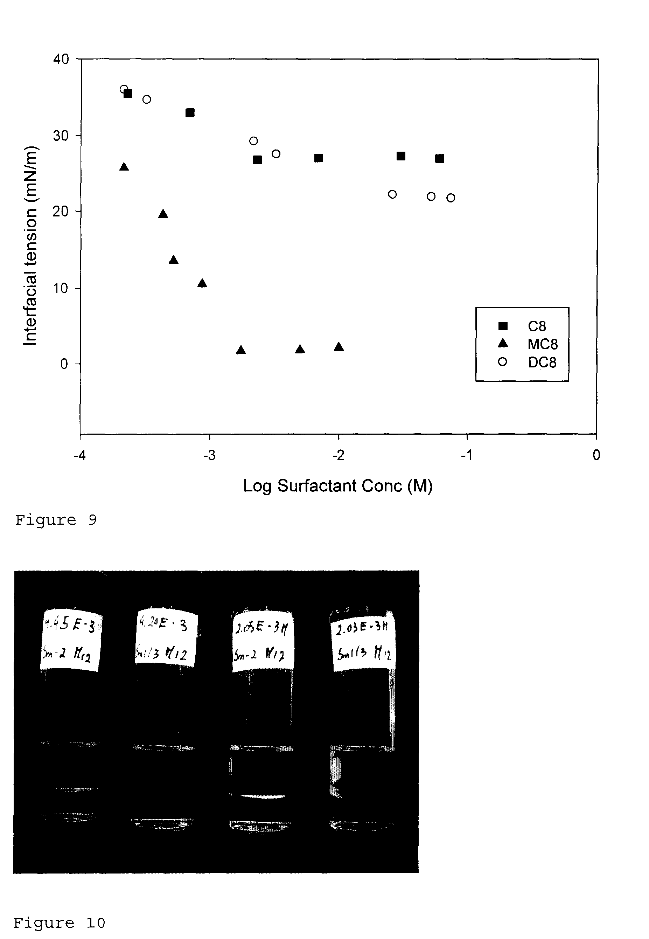

Figure 9 shows the interfacial tension properties of different surfactants. As can be observed, monocaprylin is considerably more interfacially active than the corresponding free fatty acid and diglyceride. The interfacial tension of pure Tricaprylin (free of any residues of polar lipids), should remain above 30 mN/m. Therefore, a more interfacial active molecule (like monocaprylin), has the capacity to exclude the triglyceride from the interface.

Example 4 shows the lipase reaction in 2-phase systems in the presence of a non-hydrolysable and hydrolysable monoglyceride. As can be observed even optically, the presence of non-cleavable surfactants, decrease the hydrolysis of p-nitrophenylpalmitate (the hydrolysis of p-nitrophenylpalmitate generates nitrophenol which is a chromogenic molecule) more than cleavable surfactants (figure 10).

## Claims

1. Use of a formulation comprising at least one surfactant with an interfacial pressure that is sufficiently high to control the access of lipase substrates to the interface between a lipophilic phase and a hydrophilic phase to regulate lipolysis.

2. Use in accordance with claim 1, wherein the at least one surfactant has an interfacial pressure that is sufficiently high to at least partially replace lipase substrates from an interface between a lipophilic phase and a hydrophilic phase.

3. Use in accordance with one of the preceding claims wherein lipolysis is mediated by a lipase selected from the group consisting of lingual, pancreatic and gastric lipases or combinations thereof.

4. Use in accordance with one of the preceding claims wherein the formulation further comprises a lipophilic phase and a hydrophilic phase.

5. Use in accordance with one of the preceding claims wherein the formulation comprises a lipophilic phase and a hydrophilic phase and is present in the form of an emulsion.

6. Use in accordance with one of the preceding claims wherein the emulsion has an average particle diameter of 5 nm-100 µm.

7. Use in accordance with one of the preceding claims wherein the lipophilic phase comprises at least one lipid.

8. Use in accordance with one of the preceding claims wherein the lipid is a nutritionally valuable oil.

9. Use in accordance with one of the preceding claims wherein the oil is selected from the group consisting of triglycerides, fatty acid derivatives, such as fatty acid amides, and mixtures thereof.

10. Use in accordance with one of the preceding claims wherein the at least one surfactant is at least partially located at the oil-water interface.

11. Use in accordance with one of the preceding claims wherein the surfactant has a higher affinity to the interface between the hydrophilic and lipophilic phase than the at least one lipid and is non-cleavable by lipases of the body.

12. Use in accordance with one of the preceding claims wherein the lipase is selected from the group consisting of gastro-intestinal lipases, in particular lingual lipase, gastric lipase and pancreatic lipase or mixtures thereof.

13. Use in accordance with one of the preceding claims wherein the surfactant is a food or pharmaceutical grade surfactant.

14. Use in accordance with one of the preceding claims wherein the surfactant is a mono- or di-acyl glyceride, wherein the Sn-2 position is acylated.

15. Use in accordance with one of the preceding claims wherein the fatty acid residues of the surfactants have a chain length of between 8 and 22 carbon atoms.

16. Use in accordance with one of the preceding claims wherein the fatty acid residue is selected from the group consisting of saturated and polyunsaturated fatty acid residues.

17. Use in accordance with one of the preceding claims wherein the surfactant is selected from the group consisting of low molecular weight surfactants such as myristic acid, oleic acid, lauric acid, stearic acid, palmitic acid, PEG 1-4 stearate, PEG 2-4 oleate, PEG-4 dilaurate, PEG-4 dioleate, PEG-4 distearate, PEG-6 dioleate, PEG-6 distearate, PEG-8-dioleate, PEG-3-16 castor oil, PEG 5-10 hydrogenated castor oil, PEG 6-20 corn oil, PEG 6-20 almond oil, PEG-6 olive oil, PEG-6 peanut oil, PEG-6 palm kernel oil, PEG-6 hydrogenated palm kernel oil, PEG-4 capric/caprylic triglyceride, mono, di, tri, tetraesters of vegetable oil and sorbitol, pentaerythrityl di, tetra stearate, isostearate, oleate, caprylate or caprate, polyglyceryl-3 dioleate, stearate, or isostearate, plyglyceryl 4-10 pentaoleate, polyglyceryl 2-4 oleate, stearate, or isostearate, polyglyceryl 4-10 pentaoleate, polyglycewryl-3 dioleate, polyglyceryl-6 dioleate, polyglyceryl-10 trioleate, polyglyceryl-3 distearate propylene glycol mono- or diesters of C₆ to C₂₀ fatty acid, monoglycerides of C₆ to C₂₀ fatty acid, lactic acid derivatives of monoglycerides, lactic acid dericatives of diglycerides, diacetyl tartaric ester of monoglycerides, triglycerol monostearate cholesterol, phytosterol, PEG 5-20 soya sterol, PEG-6 sorbitan tetra, hexasterarate, PEG-6 sorbitan tetraoleate, sorbitan monolaurate, sorbitan monopalmitate, sorbitan mono trioleate, sorbitan mono and tristearate, sorbitan monoisostearate, sorbitan sesquioleate, sorbitan sesquistearate, PEG-2-5 oleyl ether, POE 2-4 lauryl ether, PEG-2 cetyl ether, PEG-2 stearyl ether, sucrose ester, sucrose distearate, sucrose dipalmitate, ethyl oleate, isopropyl myristate, isopropyl palmitate, ethyl linoleate, isopropyl linoleate, poloxamers, phospolipids, lyso-phospholipids, lecithins, cephalins, oat lipids, glycolipids, and amphiphilic lipids from plants, or high molecular weight surfactants such as proteins from plant or animal origin; and mixtures thereof.

18. Use in accordance with one of the preceding claims wherein the surfactant is present in an amount of 0.1 - 99 weight-% of the formulation.

19. Use in accordance with one of the preceding claims to produce a composition.

20. Use in accordance with one of the preceding claims wherein the surfactant is present in an amount of 0.1 - 50 weight-% of the composition.

21. Use in accordance with one of claims 19-20 wherein the composition is a pharmaceutical composition or a food product.

22. Use in accordance with one of the preceding claims wherein the formulation exhibits an interfacial pressure of about 5-50 mN/m.

23. Use in accordance with one of the preceding claims in a body of a mammal, in particular of a human or a pet.

24. Use in accordance with one of the preceding claims to expel lipase substrates from an oil-water interface in the stomach, duodenum, ileum and/or jejunum.

25. Use in accordance with one of the preceding claims to reduce lipid digestion.

26. Use in accordance with one of the preceding claims to retard fat digestion.

27. Use in accordance with one of the preceding claims to decrease energy release from ingested food.

28. Use in accordance with one of the preceding claims to prolong the feeling of satiety

29. Use in accordance with one of the preceding claims to improve satiation.

30. Composition comprising at least one oil and enriched with at least one surfactant wherein the surfactant is non-cleavable by at least one lipase, has a higher affinity to the interface between the hydrophilic and lipophilic phase than the at least one lipid and is present in a weight ratio to the at least one lipid of about 1:1000 - 100:1.

31. Composition in accordance claim 30 wherein the composition further comprises a hydrophilic phase.

32. Composition in accordance with one of claims 30-31 wherein the composition is present in the form of an emulsion.

33. Composition in accordance with claim 32 wherein the emulsion has an average particle diameter of 5nm-100 µm.

34. Composition in accordance with one of claims 30-33 wherein the oil is a nutritionally valuable oil, preferably selected from the group consisting of triglycerides, fatty acid derivatives, such as fatty acid amides, and mixtures thereof.

35. Composition in accordance with one of claims 30-34 wherein the at least one surfactant is at least partially located at the oil-water interface.

36. Composition in accordance with one of claims 30-35 wherein the lipase is selected from the group consisting of gastro-intestinal lipases, in particular lingual lipase, gastric lipase and pancreatic lipase or mixtures thereof.

37. Composition in accordance with one of claims 30-36 wherein the surfactant is a food grade surfactant.

38. Composition in accordance with one of claims 30-37 wherein the surfactant is a mono- or di-acyl glyceride, wherein the Sn-2 position is acylated.

39. Composition in accordance with one of claims 30-38 wherein the fatty acid residues of the surfactant have a chain length of between 8 and 22 carbon atoms.

40. Composition in accordance with one of claims 30-39 wherein the composition exhibits an interfacial pressure of about 5-50 mN/m.

41. Composition in accordance with one of claims 30-40 wherein the surfactant is selected from the group consisting low molecular weight surfactants such as myristic acid, oleic acid, lauric acid, stearic acid, palmitic acid, PEG 1-4 stearate, PEG 2-4 oleate, PEG-4 dilaurate, PEG-4 dioleate, PEG-4 distearate, PEG-6 dioleate, PEG-6 distearate, PEG-8-dioleate, PEG-3-16 castor oil, PEG 5-10 hydrogenated castor oil, PEG 6-20 corn oil, PEG 6-20 almond oil, PEG-6 olive oil, PEG-6 peanut oil, PEG-6 palm kernel oil, PEG-6 hydrogenated palm kernel oil, PEG-4 capric/caprylic triglyceride, mono, di, tri, tetraesters of vegetable oil and sorbitol, pentaerythrityl di, tetra stearate, isostearate, oleate, caprylate or caprate, polyglyceryl-3 dioleate, stearate, or isostearate, plyglyceryl 4-10 pentaoleate, polyglyceryl 2-4 oleate, stearate, or isostearate, polyglyceryl 4-10 pentaoleate, polyglycewryl-3 dioleate, polyglyceryl-6 dioleate, polyglyceryl-10 trioleate, polyglyceryl-3 distearate propylene glycol mono- or diesters of C₆ to C₂₀ fatty acid, monoglycerides of C₆ to C₂₀ fatty acid, lactic acid derivatives of monoglycerides, lactic acid dericatives of diglycerides, diacetyl tartaric ester of monoglycerides, triglycerol monostearate cholesterol, phytosterol, PEG 5-20 soya sterol, PEG-6 sorbitan tetra, hexasterarate, PEG-6 sorbitan tetraoleate, sorbitan monolaurate, sorbitan monopalmitate, sorbitan mono trioleate, sorbitan mono and tristearate, sorbitan monoisostearate, sorbitan sesquioleate, sorbitan sesquistearate, PEG-2-5 oleyl ether, POE 2-4 lauryl ether, PEG-2 cetyl ether, PEG-2 stearyl ether, sucrose ester, sucrose distearate, sucrose dipalmitate, ethyl oleate, isopropyl myristate, isopropyl palmitate, ethyl linoleate, isopropyl linoleate, poloxamers, phospolipids, lyso-phospholipids, lecithins, cephalins, oat lipids, glycolipids, and amphiphilic lipids from plants, or high molecular weight surfactants such as proteins from plant or animal origin; and mixtures thereof.

42. Composition in accordance with one of claims 30-41 wherein the composition is a pharmaceutical composition or a food product.
